# EUROPEAN PATENT APPLICATION

(11) **EP 4 775 234 A1**
(43) Date of publication of application: **15.07.2026**
(21) Application number: 24871854.6
(22) Date of filing: 11.09.2024
(51) Int. Cl.: A61M 5/14, A61B 17/34, A61M 31/00

(54) **MEDICAL DEVICE**

(30) Priority: 29.09.2023 JP 2023169994
(71) Applicant: Terumo Kabushiki Kaisha, Tokyo 151-0072 (JP)
(72) Inventor: HAZAMA, Kenichi, Ashigarakami-gun, Kanagawa 259-0151 (JP); KAKIMOTO, Yasuhiro, Ashigarakami-gun, Kanagawa 259-0151 (JP)
(74) Representative: TBK
(86) International application number: PCT/JP2024/032504
(87) International publication number: WO 2025/070057

(57) **Abstract**

Provided is a medical device capable of preventing an excessive clearance from being formed with an inner wall of a delivery device when a puncture device is delivered to a target site in a living body, and capable of easily and smoothly performing protrusion of the puncture device from a first lumen of an outer sheath and accommodation of the puncture device into the first lumen of the outer sheath by operating forward and backward movement of a hand handle portion.

A medical device 10 is configured to be retractably inserted into a channel 511 of an insertion portion 510 of an endoscope device 500, and includes a puncture device 100, an outer sheath 200 into which the puncture device is inserted, and a hand handle portion 300 disposed on a proximal end side of the puncture device and the outer sheath. The hand handle portion includes a first connection portion 320 connected to the outer sheath, and a second connection portion 330 connected to at least a part of the puncture device and relatively moving forward and backward with respect to the first connection portion to cause a distal end of the puncture device to protrude from a distal end of the outer sheath.

## Description

### Technical Field

The present invention relates to a medical device.

### Background Art

In the medical field, development of a medical device used for puncture of a living tissue and administration of a drug solution to a target site in a living body has been advanced.

In the development of the medical device as described above, various attempts have been made to realize a more efficient procedure. As one of such approaches, for example, a structure similar to that of a conventional biopsy needle from the viewpoint of securing puncture performance for a living tissue can be adopted. However, the biopsy needle tends to have a large outer diameter in order to achieve the original purpose such as collection of a tissue piece. Therefore, in a case where a structure similar to that of the biopsy needle is adopted in the above-described medical device, an increase in bleeding risk at the time of administration of a drug solution and leakage of the drug solution become problems.

As a measure against the above problems, for example, the outer diameter of the device can be configured to be smaller than the outer diameter of a general biopsy needle. However, in a case where the outer diameter of the device is reduced, an excessive clearance may be generated between the medical device having a reduced outer diameter and the inner wall of a delivery device when performing a procedure using the delivery device such as an endoscope device in combination. When such an excessive clearance is generated, the medical device may be twisted or deflected in the course of traveling through a large number of bent portions in the living body due to the fact that the delivery device and the medical device have a relatively long length. In addition, when the medical device is twisted or deflected as described above, a positional deviation in the radial direction with respect to the central axis of the lumen (channel) of the delivery device occurs between the proximal portion (hand portion) of the medical device and the distal portion (blade edge portion) of the medical device. As a result, it can be difficult to ascertain a position of the distal portion of the medical device from a diagnostic image (for example, an ultrasound image acquired by an ultrasound endoscope), the operability of the medical device deteriorates (torque transmission is not sufficient), the pushability of the medical device deteriorates, and the pushing force applied on the proximal portion side cannot be sufficiently transmitted to the distal portion side.

In relation to the above problems, Patent Literature 1 below discloses a multi-lumen drug solution administration device including a catheter body having flexibility to be inserted into a living body, a needle portion movable forward and backward in a lumen of the catheter body, and a connector portion disposed at a proximal portion of the catheter body and connected to the needle portion.

In the procedure using the drug solution administration device of Patent Literature 1, after the catheter body is delivered to a peripheral portion of a target site in the living body, the needle portion is caused to protrude toward the living tissue through a hole portion (side hole) provided in the catheter body. An operator (a medical operator such as a doctor) punctures a living tissue with the needle portion and then operates a syringe connected to the connector portion to administer a drug solution to the target site.

### Citation List

### Patent Literature

Patent Literature 1: JP 2004-329487 A

### Summary of Invention

### Technical Problem

In the drug solution administration device of Patent Literature 1, the catheter body accommodating the needle portion has an outer diameter larger than that of the needle portion. Therefore, it is considered that the occurrence of various problems due to the excessive clearance generated between the medical device and the inner wall of the delivery device described above can be prevented in advance. However, the drug solution administration device of Patent Literature 1 has the following problems.

In the procedure using the drug solution administration device of Patent Literature 1, as described above, after the needle portion is delivered to a predetermined position in the living body via the elongated catheter body, an operation of causing the needle portion to protrude toward the target site is performed. Therefore, after delivering the needle portion together with the catheter body to the vicinity of the target site, the operator needs to appropriately and precisely control forward and backward movement of the needle portion.

In a procedure including a treatment of puncturing a living tissue with a needle portion, it is desirable that puncture of the needle portion with respect to the living tissue and removal of the needle portion from the living tissue proceed as quickly and smoothly as possible in consideration of a burden on a patient. However, in the medical device of Patent Literature 1, a structure and a device for moving the needle portion forward and backward in the lumen of the catheter body are not particularly considered. For example, in a procedure using the drug solution administration device of Patent Literature 1, when puncturing a living tissue with the needle portion or removing the needle portion from the living tissue, it is necessary for an operator to directly hold the proximal portion of the needle portion extending from the connector portion disposed outside the living body with fingers or the like and perform an operation of moving the entire needle portion forward and backward together with the proximal portion of the needle portion. Therefore, in the procedure using the medical device of Patent Literature 1, it is not easy to smoothly perform puncture of the needle portion with respect to the living tissue and removal of the needle portion from the living tissue.

The present invention has been made in view of the above problems, and it is an object of the present invention to provide a medical device capable of preventing an excessive clearance from being formed with an inner wall of a delivery device when a puncture device is delivered to a target site in a living body, and capable of easily and smoothly performing protrusion of the puncture device from a first lumen of an outer sheath and accommodation of the puncture device into the first lumen of the outer sheath by operating forward and backward movement of a hand handle portion.

### Solution to Problem

The present invention is achieved by any one of the following means (1) to (9).
(1) A medical device that is retractably inserted into a delivery device, the medical device including a puncture device, an outer sheath into which the puncture device is inserted, and a hand handle portion disposed on a proximal end side of the puncture device and the outer sheath, in which the outer sheath includes a first surface defining a first lumen into which the puncture device is insertable inside, and a second surface defining at least one second lumen between the first surface and the second surface, and the hand handle portion includes a first connection portion connected to the outer sheath, and a second connection portion connected to at least a part of the puncture device, and relatively moving forward and backward with respect to the first connection portion to cause a distal end of the puncture device to protrude from a distal end of the outer sheath.
(2) The medical device according to (1), in which the puncture device includes a puncture needle including a blade edge portion and a needle tube portion, and an inner sheath including a third lumen into which the puncture needle is retractably inserted.
(3) The medical device according to (2), in which the inner sheath is connected to the hand handle portion at the second connection portion, and at least a part of the hand handle portion further includes a third connection portion that is located further toward the proximal end side than the second connection portion, to which a proximal portion of the puncture needle is connected, and that relatively moves forward and backward with respect to the second connection portion to cause the blade edge portion to protrude from a distal end of the inner sheath.
(4) The medical device according to any one of (1) to (3), in which the puncture needle has a puncture lumen that allows a fluid to be delivered to a distal end side.
(5) The medical device according to (4), further including a first port that is disposed to communicate with the puncture lumen via the third connection portion of the hand handle portion and allows a fluid to be supplied into the puncture lumen.
(6) The medical device according to any one of (1) to (5), in which the first connection portion includes a second port that is disposed to communicate with inside of the first lumen of the outer sheath and enables fluid communication inside and outside the first lumen.
(7) The medical device according to any one of (1) to (6), in which the outer sheath includes an inner tube having the first surface and the first lumen, an outer tube having the second surface and the second lumen, and a fixing member disposed between the inner tube and the outer tube and fixing the inner tube and the outer tube.
(8) The medical device according to (7), in which the inner tube and the outer tube are concentrically disposed by the fixing member.
(9) The medical device according to (8), in which the fixing member extends spirally around central axes of the inner tube and the outer tube.

### Advantageous Effects of Invention

According to the medical device according to (1), it is possible to prevent an excessive clearance from being formed with the inner wall of the delivery device when the puncture device is delivered to the target site in the living body. In addition, according to the medical device according to (1), the protrusion of the puncture device from the first lumen of the outer sheath and the accommodation of the puncture device into the first lumen of the outer sheath can be easily and smoothly performed by operating forward and backward movement of the hand handle portion.

### Brief Description of Drawings

[Fig. 1] Fig. 1 is a perspective view of a medical device according to an embodiment.
[Fig. 2] Fig. 2 is a perspective view of the medical device according to the embodiment.
[Fig. 3] Fig. 3 is a partial cross-sectional view in an axial direction of the medical device according to the embodiment.
[Fig. 4] Fig. 4 is a partial cross-sectional view illustrating a part of the medical device according to the embodiment in an enlarged manner.
[Fig. 5] Fig. 5 is a partial cross-sectional view illustrating a part of the medical device according to the embodiment in an enlarged manner.
[Fig. 6] Fig. 6 is an axis-orthogonal cross-sectional view of the medical device taken along an arrow 6A-6A in Fig. 3.
[Fig. 7] Fig. 7 is a perspective view illustrating the vicinity of a distal portion of a puncture device.
[Fig. 8] Fig. 8 is a perspective view illustrating the vicinity of the distal portion of the puncture device.
[Fig. 9] Fig. 9 is a perspective view illustrating the vicinity of the distal portion of the puncture device.
[Fig. 10] Fig. 10 is a cross-sectional view taken along an axial direction of the distal portion of the puncture device.
[Fig. 11] Fig. 11 is a perspective cross-sectional view in the axial direction of the distal portion of the puncture device.
[Fig. 12] Fig. 12 is a plan view of a puncture needle.
[Fig. 13] Fig. 13 is a front view of the puncture needle.
[Fig. 14] Fig. 14 is a cross-sectional view schematically illustrating a usage example of the medical device according to the embodiment.
[Fig. 15] Fig. 15 is a cross-sectional view schematically illustrating a usage example of the medical device according to the embodiment.
[Fig. 16] Fig. 16 is a cross-sectional view schematically illustrating a usage example of the medical device according to the embodiment.
[Fig. 17] Fig. 17 is a cross-sectional view schematically illustrating a usage example of the medical device according to the embodiment.
[Fig. 18] Fig. 18 is a cross-sectional view schematically illustrating a usage example of the medical device according to the embodiment.
[Fig. 19] Fig. 19 is a partial cross-sectional view in an axial direction of a medical device according to Modification 1.
[Fig. 20] Fig. 20 is an enlarged perspective view illustrating a part of an outer sheath according to Modification 2.
[Fig. 21] Fig. 21 is an axis-orthogonal cross-sectional view of the outer sheath taken along an arrow 21A-21A in Fig. 20.
[Fig. 22] Fig. 22 is a partial cross-sectional view in an axial direction of a medical device according to Modification 3.

### Description of Embodiments

Hereinafter, an embodiment of the present invention will be described with reference to the drawings. Dimensional ratios in the drawings are exaggerated for convenience of description, and may be different from actual ratios.

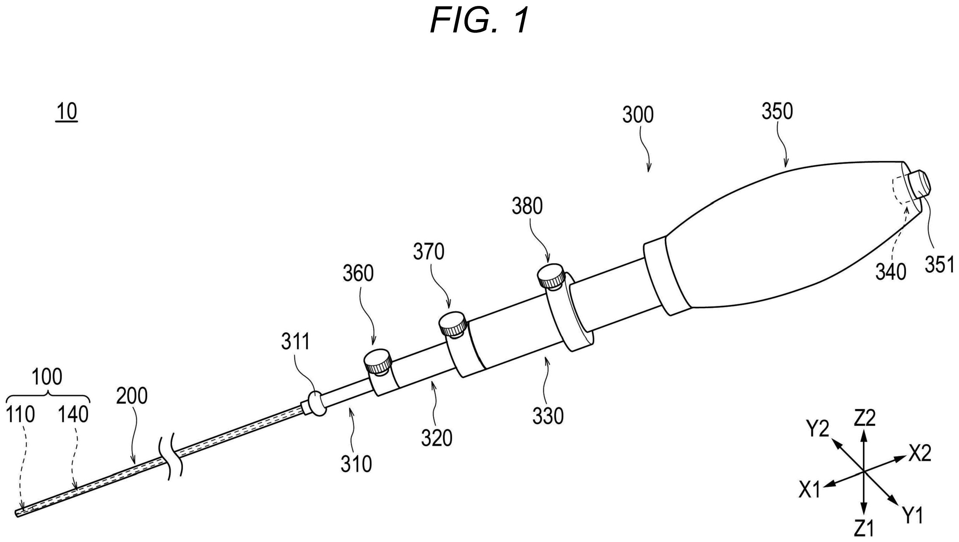

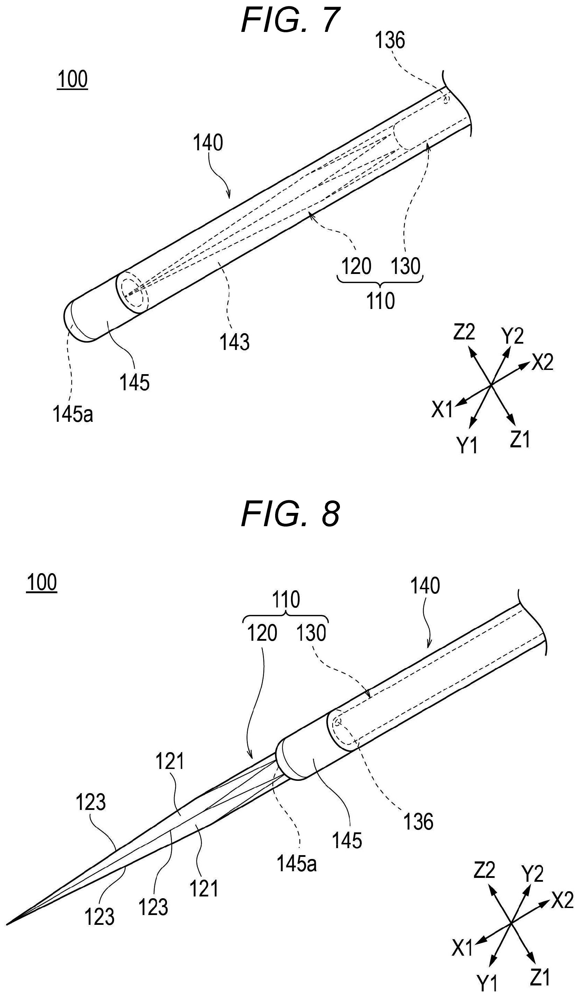

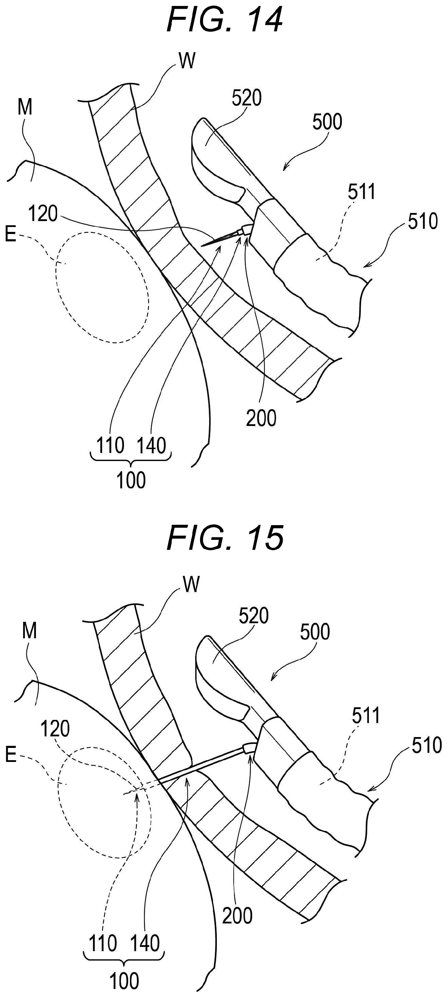

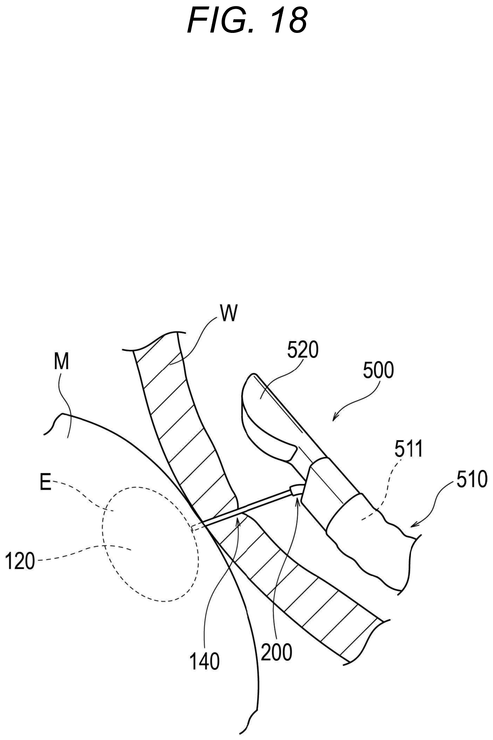

Figs. 1 to 6 are diagrams illustrating an overall configuration of a medical device 10 according to an embodiment, Figs. 7 to 13 are diagrams for describing a puncture device 100 according to an embodiment, and Figs. 14 to 18 are diagrams for describing a procedure using the medical device 10.

In the present specification, each direction regarding the medical device 10 is defined as follows. The side of the medical device 10 to be introduced into a living body (the side indicated by an arrow X1 in the drawing) is defined as a distal end side, and an end portion located on the distal end side in each member and each constituent is defined as a "distal portion". In addition, the side on which a grip portion 350 of a hand handle portion 300 of the medical device 10 is disposed (the side indicated by an arrow X2 in the drawing) is defined as a proximal end side, and an end portion located on the proximal end side in each member and each constituent is defined as a "proximal portion". Further, a direction from the distal portion to the proximal portion (or from the proximal portion to the distal portion) is referred to as an "axial direction (a direction indicated by arrows X1-X2)". Arrows Y1-Y2 in each drawing indicate a width direction orthogonal to the axial direction, and arrows Z1-Z2 indicate a height direction orthogonal to each of the axial direction and the width direction.

### (Medical Device 10)

As illustrated in Figs. 14 to 18, the medical device 10 is configured as a medical device that is retractably inserted into a delivery device.

In the present embodiment, an endoscope device 500 known in the medical field is exemplified as the delivery device. When the medical device 10 is delivered to a target site E in a living body, the medical device 10 can be inserted into a channel 511 of an insertion portion 510 included in the endoscope device 500 in a retractable manner. The endoscope device 500 can be, for example, a known ultrasound endoscope in which an ultrasound imaging portion 520 is disposed at the distal portion of the insertion portion 510. However, specific structures, product specifications, product types, and the like of the endoscope device 500 are not particularly limited.

As illustrated in Figs. 1 to 3, the medical device 10 includes a puncture device 100, an outer sheath 200 into which the puncture device 100 is inserted, and a hand handle portion 300 disposed on the proximal end side of the puncture device 100 and the outer sheath 200.

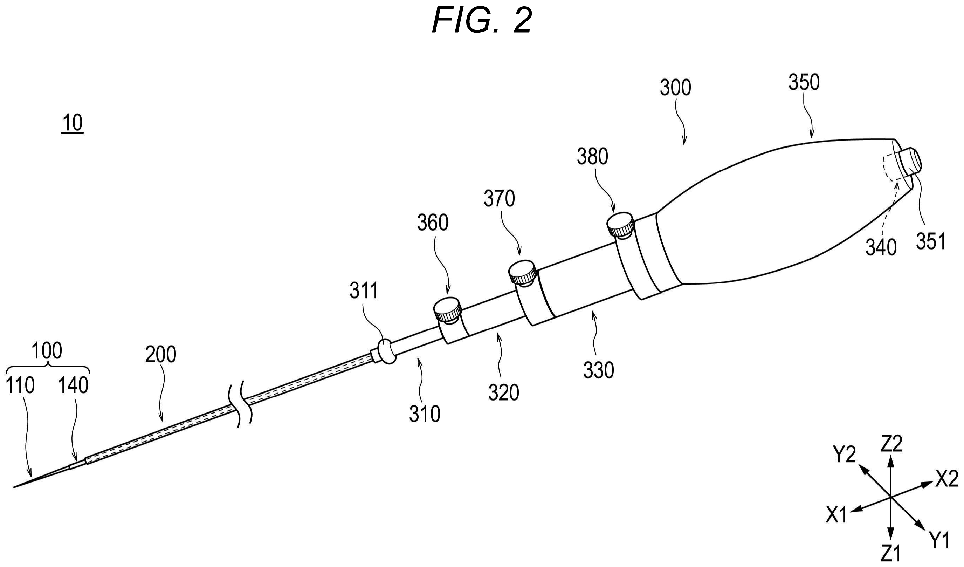

In the medical device 10, the puncture device 100, the inner sheath 140, and the outer sheath 200 are connected to each part of the hand handle portion 300, and relative positions in the axial direction between the three constituent members can be changed and adjusted by operating the hand handle portion 300. Note that Fig. 1 exemplifies a state in which the inner sheath 140 and the puncture device 100 are accommodated in the outer sheath 200, and Fig. 2 exemplifies a state in which the inner sheath 140 and the puncture device 100 protrude toward the distal end side of the outer sheath 200.

### (Outer Sheath 200)

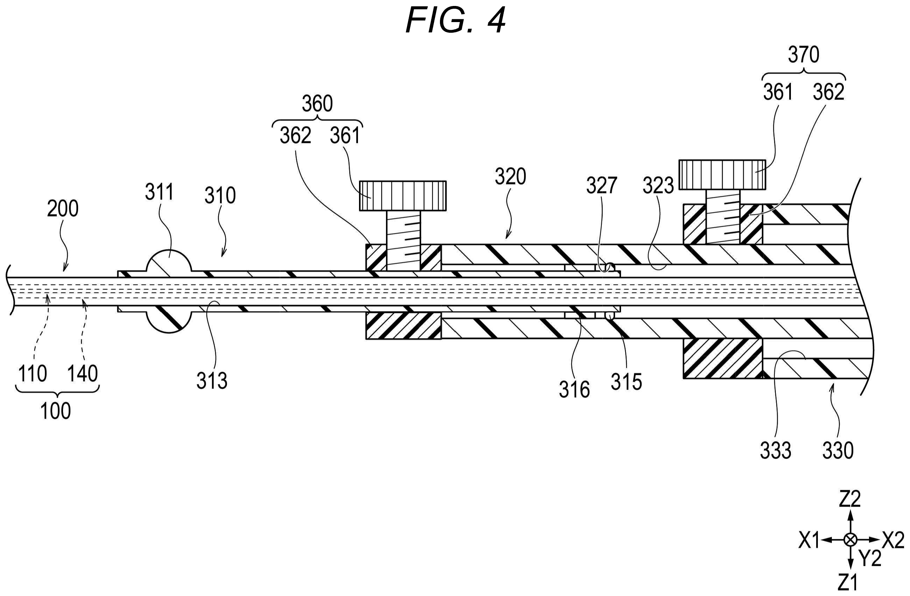

As illustrated in Figs. 4, 5, and 6, the outer sheath 200 includes a member having a multi-tube structure having predetermined lumens 203 and 204 in the outer sheath 200.

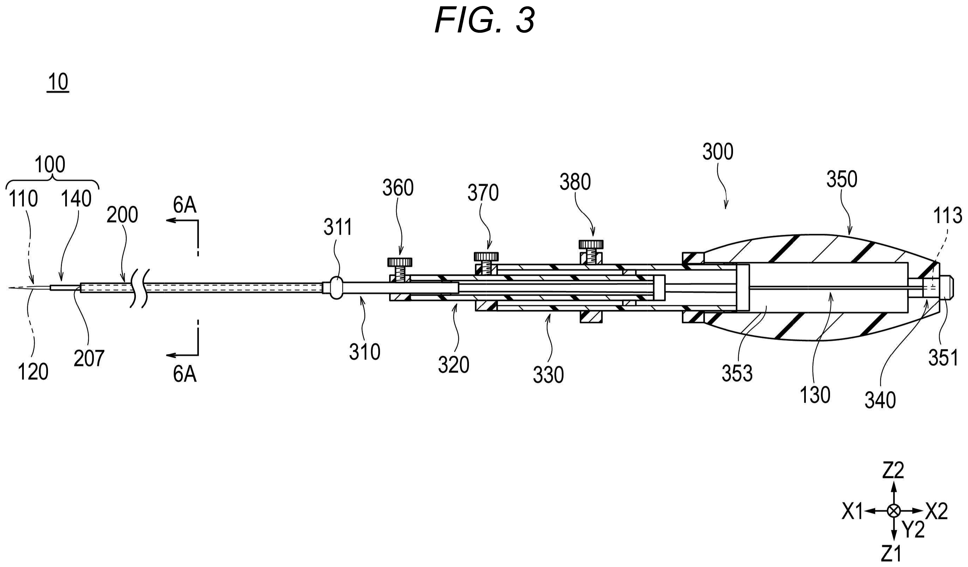

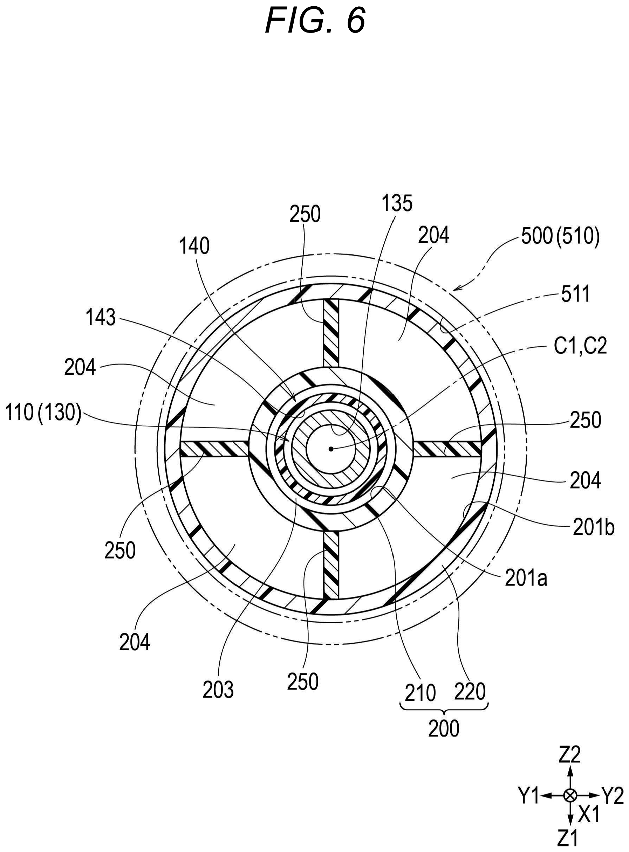

As illustrated in Fig. 6, the outer sheath 200 has a first surface 201a defining a first lumen 203 into which the puncture device 100 can be inserted inside, and a second surface 201b defining at least one second lumen 204 between the first surface 201a and the second surface 201b. Fig. 6 is an axis-orthogonal cross-sectional view taken along arrow 3A-3A in Fig. 3.

The outer sheath 200 includes an inner tube 210 having the first surface 201a and the first lumen 203, an outer tube 220 having the second surface 201b and the second lumen 204, and a fixing member 250 that is disposed between the inner tube 210 and the outer tube 220 and fixes the inner tube 210 and the outer tube 220.

The first surface 201a is formed of an inner surface of the inner tube 210. The second surface 201b is formed of an inner surface of the outer tube 220. The first lumen 203 is formed inside the inner tube 210 and extends over substantially the entire length in the axial direction of the inner tube 210. The second lumen 204 is defined between the outer surface of the inner tube 210 and the inner surface of the outer tube 220, and extends over substantially the entire axial length of the inner tube 210 and the outer tube 220.

As illustrated in Fig. 6, in the present embodiment, four second lumens 204 are evenly (axially symmetrically) formed in the outer tube 220 in the circumferential direction. In addition, the respective second lumens 204 are partitioned by the fixing member 250 to have substantially the same cross-sectional area at different positions in the circumferential direction with reference to central axes C1 and C2 of the inner tube 210 and the outer tube 220. Therefore, the second lumen 204 is defined by the outer surface of the inner tube 210, the inner surface of the outer tube 220, and the exposed surface of the fixing member 250. The number of the second lumens 204 is not particularly limited.

The fixing member 250 fixes the inner tube 210 and the outer tube 220 to maintain a relative positional relationship between the inner tube 210 and the outer tube 220 (a positional relationship on an axis-orthogonal cross-sectional view illustrated in Fig. 6).

In the present embodiment, as illustrated in Fig. 6, the inner tube 210 and the outer tube 220 are concentrically disposed by the fixing member 250. That is, in the axis-orthogonal cross-sectional view illustrated in Fig. 6, the central axis C1 of the inner tube 210 and the central axis C2 of the outer tube 220 are disposed at positions overlapping each other.

As illustrated in Fig. 6, four fixing members 250 are disposed at equal intervals in the circumferential direction of the outer sheath 200 with respect to the central axis (same as central axes C1 and C2) of the outer sheath 200. The circumferential angle difference between the fixing members 250 is approximately 90°.

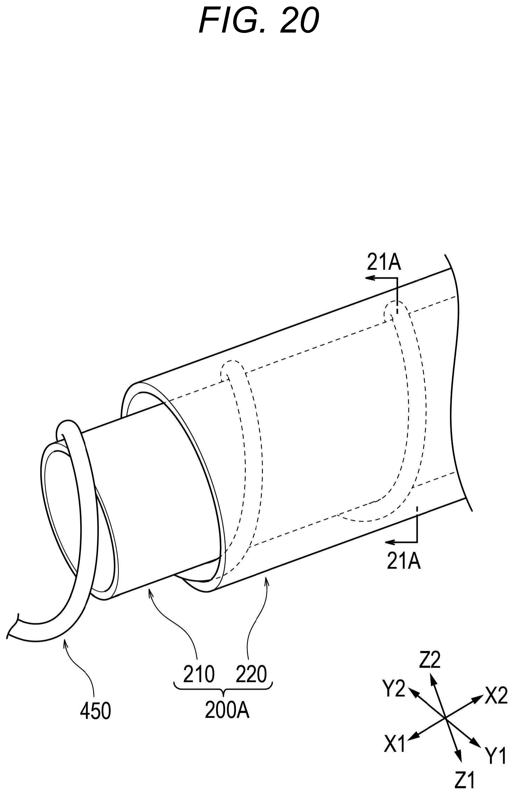

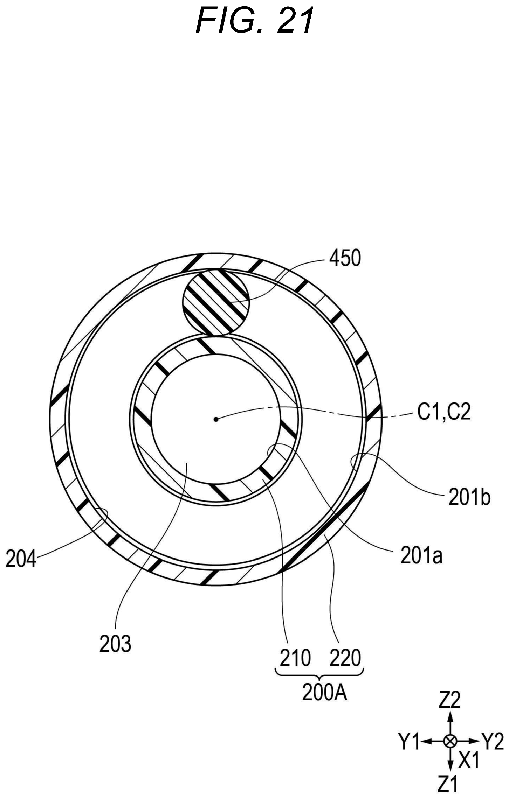

The number, disposition, a cross-sectional shape (the cross-sectional shape illustrated in Fig. 6), and the like of the fixing members 250 are not particularly limited as long as the fixing members exert a function of fixing the inner tube 210 and the outer tube 220. For example, as described in a modification that will be described later, the fixing member may be formed of a single member extending in the axial direction in a spiral shape (see Figs. 20 and 21).

As illustrated in Fig. 3, a distal end opening 207 that allows the puncture device 100 to protrude from the outer sheath 200 is provided at the distal end of the outer sheath 200.

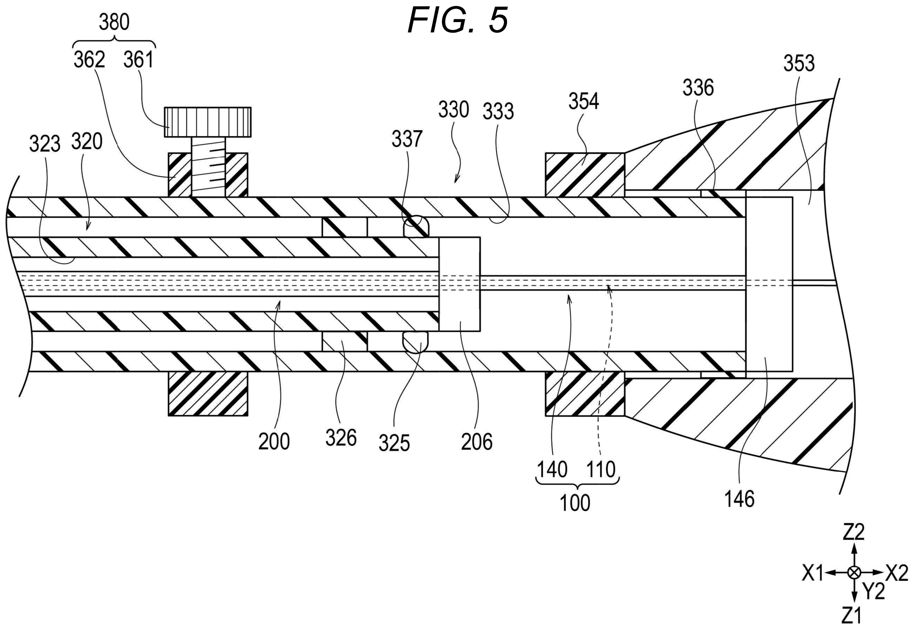

As illustrated in Fig. 5, the proximal portion 206 of the outer sheath 200 is connected to a first connection portion 320 that will be described later. Therefore, when the first connection portion 320 is moved forward and backward in the axial direction, the outer sheath 200 moves forward and backward in the axial direction in conjunction with the forward and backward movement of the first connection portion 320. The proximal portion of the outer sheath 200 indicates the proximal portion of the inner tube 210 and the proximal portion of the outer tube 220.

The outer sheath 200 (the inner tube 210 and the outer tube 220) and the inner sheath 140 that will be described later may be made of, for example, a material similar to a material used as a constituent material of a catheter or the like known in the medical field. Examples of the constituent material of the outer sheath 200 and the inner sheath 140 can include polyolefins such as polyethylene, polypropylene, an ethylene-propylene copolymer, polyether ether ketone, polyimide, and an ethylene-vinyl acetate copolymer, thermoplastic resins such as soft polyvinyl chloride, various elastomers such as a polyurethane elastomer, a polyamide elastomer, and a polyester elastomer, and crystalline plastics such as polyamide, crystalline polyethylene, and crystalline polypropylene.

The fixing member 250 is preferably made of a relatively flexible resin material not to excessively increase the rigidity of the outer sheath 200. Examples of the material of the fixing member 250 can include polyolefin (for example, polyethylene, polypropylene, polybutene, an ethylene-propylene copolymer, an ethylene-vinyl acetate copolymer, an ionomer, or a mixture of two or more kinds of the polyolefin materials listed), polyvinyl chloride, polyamide, polyamide elastomer, polyurethane, polyurethane elastomer, and polyimide.

### (Puncture Device 100)

As illustrated in Figs. 3 and 7 to 9, the puncture device 100 includes a puncture needle 110 including a blade edge portion 120 and a needle tube portion 130, and an inner sheath 140 including a third lumen 143 into which the puncture needle 110 is retractably inserted.

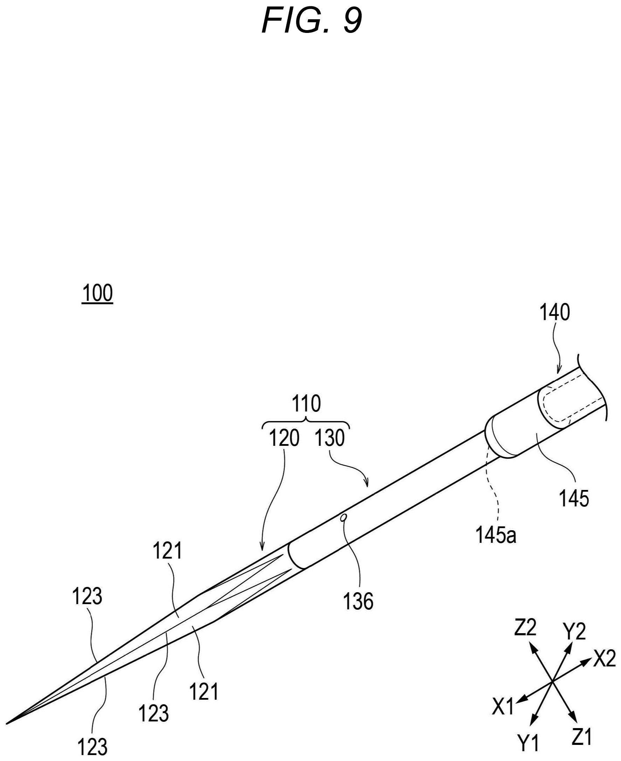

Fig. 7 illustrates a state in which a blade edge portion 120 and a needle tube portion 130 are accommodated in the third lumen 143 of the inner sheath 140. Fig. 8 illustrates a state in which the blade edge portion 120 protrudes from the third lumen 143 of the inner sheath 140. Fig. 9 illustrates a state in which the vicinity of the distal portions of the blade edge portion 120 and the needle tube portion 130 is caused to protrude from the third lumen 143 of the inner sheath 140.

As illustrated in Figs. 8, 9, and 10, a distal end member 145 is attached to the distal portion of the inner sheath 140. The distal end member 145 may be made of, for example, metal to which X-ray contrast property is added. Alternatively, the distal end member 145 may be provided with a groove, a recess, or a protrusion to have high ultrasonic reflectance. When the distal end member 145 is made of metal or the like to which X-ray contrast property is added, the distal end position of the inner sheath 140 (the position of the distal end member 145) can be visually recognized and ascertained under X-ray fluoroscopy. When the distal end member 145 is configured to have high ultrasonic reflectance, the distal end position of the inner sheath 140 (the position of the distal end member 145) can be visually recognized and ascertained by an ultrasonic device. A similar effect can be obtained by providing an X-ray opaque marker or an ultrasonic marker on the distal end member 145. A distal end opening 145a that allows the puncture needle 110 to protrude is provided at the distal end of the distal end member 145.

As illustrated in Fig. 5, the proximal portion 146 of the inner sheath 140 is connected to the hand handle portion 300 at a second connection portion 330 that will be described later. Therefore, when the second connection portion 330 is moved forward and backward in the axial direction, the inner sheath 140 moves forward and backward in the axial direction in conjunction with the forward and backward movement of the second connection portion 330. Here, the proximal portion 146 of the inner sheath 140 may include a tubular member capable of connecting the inner sheath 140 in the lumen, and a cylindrical member through which the tubular member penetrates to be fixed.

As illustrated in Fig. 5, the proximal portion 146 of the inner sheath 140 connected to the second connection portion 330 is disposed further toward the proximal end side than the proximal portion 206 of the outer sheath 200 connected to the first connection portion 320. The inner sheath 140 is inserted into the first lumen 203 (see Fig. 6) from a position located further toward the proximal end side than the proximal portion 206 of the outer sheath 200.

As illustrated in Fig. 3, a proximal portion 113 of the puncture needle 110 (a proximal portion of the needle tube portion 130) is connected to a third connection portion 340 that will be described later. The third connection portion 340 is connected to a grip portion 350 that will be described later. Therefore, when the third connection portion 340 is moved forward and backward in the axial direction together with the grip portion 350, the puncture needle 110 moves forward and backward in the axial direction in conjunction with the forward and backward movement of the third connection portion 340.

As illustrated in Figs. 3 and 5, the proximal portion 113 of the puncture needle 110 connected to the third connection portion 340 is disposed further toward the proximal end side than the proximal portion 146 of the inner sheath 140 connected to the second connection portion 330. The puncture needle 110 is inserted into the third lumen 143 (see Fig. 6) from a position located further toward the proximal end side than the proximal portion 146 of the inner sheath 140.

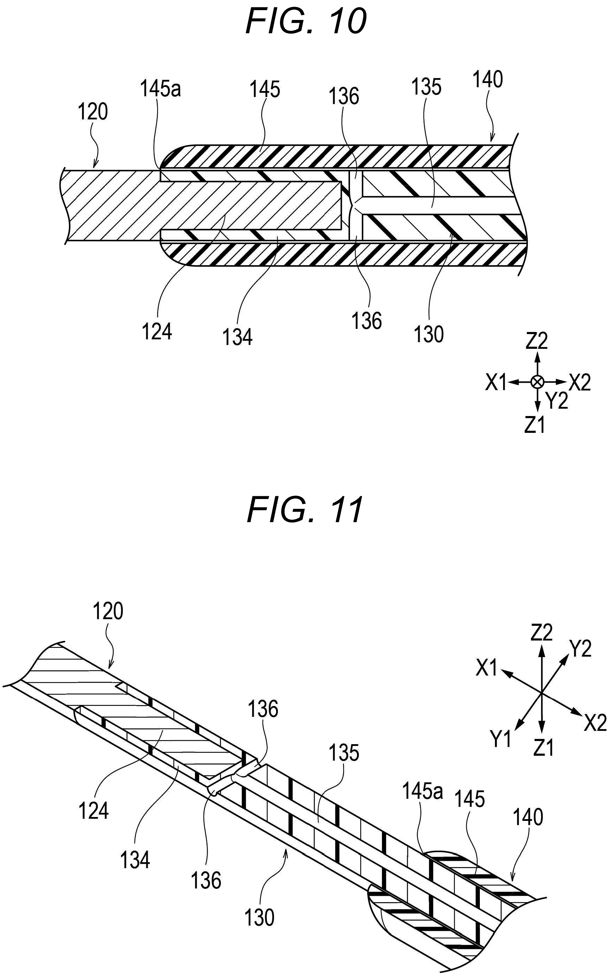

As illustrated in Figs. 10 and 11, the puncture needle 110 has a puncture lumen 135 that allows a fluid to be delivered to the distal end side.

In the present embodiment, the puncture lumen 135 of the puncture needle 110 is formed only in the needle tube portion 130. The blade edge portion 120 of the puncture needle 110 has a solid structure in which the puncture lumen 135 is not formed.

As illustrated in Fig. 3, the puncture needle 110 has a proximal end opening (not illustrated) formed in the proximal portion 113. The proximal end opening of the puncture needle 110 is disposed to communicate with a first port 351 integrally connected to the third connection portion 340. When a predetermined drug solution supply device (for example, a syringe pump known in the medical field) is connected to the first port 351 and a drug solution is supplied, the drug solution flows into the puncture lumen 135 via the first port 351 and the proximal end opening of the puncture needle 110. Note that the inflow of the drug solution into the puncture lumen 135 may be performed via a hole portion 136 that will be described later. That is, by connecting an intake-capable instrument to the first port 351 and sucking the instrument in a state in which the hole portion 136 protrudes from the inner sheath 140 (a state in which the hole portion 136 is not covered by the inner sheath 140), the drug solution may flow into the puncture lumen 135 via the hole portion 136.

As illustrated in Figs. 10 and 11, the puncture lumen 135 is connected to a hole portion 136 formed in the vicinity of the distal portion of the needle tube portion 130. When the drug solution flowing into the puncture lumen 135 reaches the vicinity of the distal portion of the needle tube portion 130 after moving to the distal end side in the axial direction along the puncture lumen 135, the drug solution is released to the outside of the needle tube portion 130 (the outside of the puncture needle 110) via the hole portion 136. Since the puncture device 100 is inserted into the third lumen 143 of the inner sheath 140 to be movable forward and backward, it is possible to prevent leakage of the drug solution from the hole portion 136 of the puncture device 100 in a state in which the blade edge portion 120 and the needle tube portion 130 are accommodated in the third lumen 143 of the inner sheath 140.

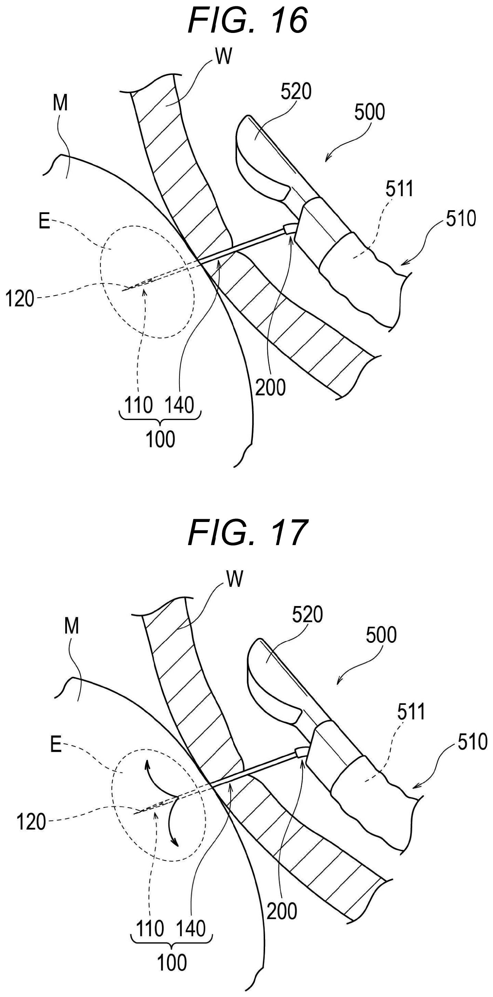

In the procedure using the medical device 10, as illustrated in Fig. 9, by supplying the drug solution to the puncture lumen 135 in a state in which the hole portion 136 formed in the needle tube portion 130 protrudes from the inner sheath 140 (a state in which the hole portion 136 is not covered by the inner sheath 140), the drug solution can be locally administered to the target site E (see Fig. 17) via the hole portion 136.

As illustrated in Figs. 10 and 11, in the present embodiment, the puncture needle 110 includes two members such as the blade edge portion 120 and the needle tube portion 130.

The blade edge portion 120 includes a first fixing portion 124 provided at the proximal portion of the blade edge portion 120. The needle tube portion 130 has a second fixing portion 134 provided at the distal portion of the needle tube portion 130.

The second fixing portion 134 has a recessed space into which the first fixing portion 124 can be fitted. The blade edge portion 120 is connected to the needle tube portion 130 by fitting the first fixing portion 124 to the second fixing portion 134. The connection between the first fixing portion 124 and the second fixing portion 134 can be appropriately reinforced by an adhesive, welding, or the like.

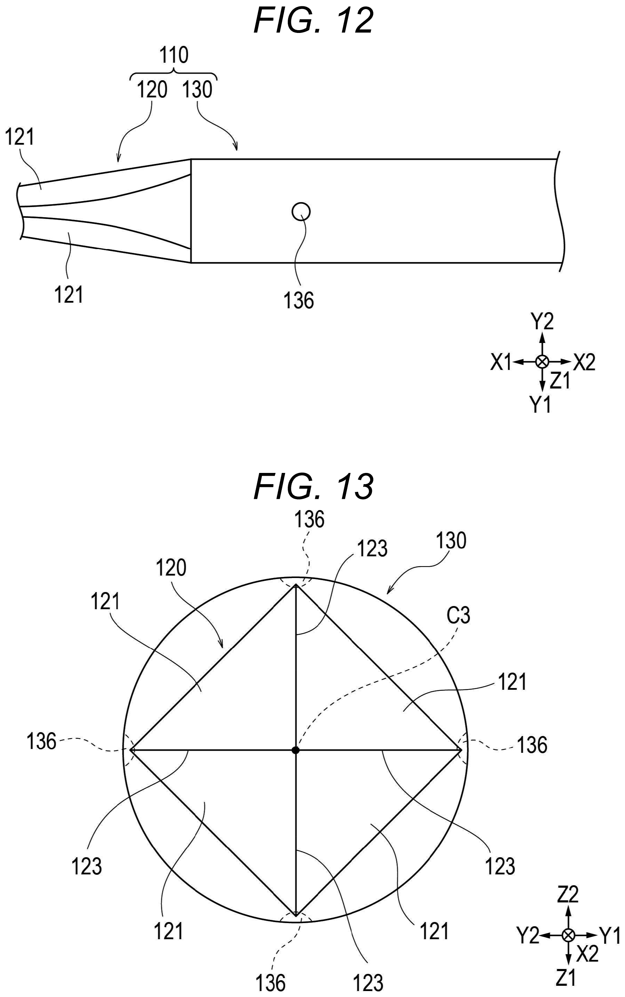

As illustrated in Fig. 13, the blade edge portion 120 has four blade surfaces 121 and four ridge lines 123 located at boundaries between the blade surfaces 121.

Note that a specific structure (for example, the number and shape of the blade surfaces 121 and the number and shape of the ridge lines 123) of the blade edge portion 120 is not particularly limited. For example, the blade edge portion 120 may have a hollow structure in which a lumen for allowing the drug solution to flow through the inside of the blade edge portion 120 is formed, or may have a distal end opening that allows the drug solution to be released from the distal end of the blade edge portion 120. In addition, the blade edge portion 120 and the needle tube portion 130 may be formed of a single integrated member.

The hole portion 136 formed in the needle tube portion 130 can be formed in a substantially circular shape in a plan view illustrated in Fig. 12, for example. Furthermore, for example, as illustrated in Fig. 13, four hole portions 136 may be provided at different positions in the circumferential direction with reference to the central axis C3 of the puncture needle 110 at equal angles (approximately 90°). However, a shape and the number of the hole portions 136, a position where the hole portion 136 is disposed in the needle tube portion 130, and the like are not particularly limited.

The puncture needle 110 (the blade edge portion 120 and the needle tube portion 130) may be formed of, for example, a metal needle containing metal as a constituent material. As the metal contained in the puncture needle 110, for example, stainless steel, aluminum, an aluminum alloy, titanium, a titanium alloy, or other metals may be used.

### (Hand Handle Portion 300)

As illustrated in Figs. 3, 4, and 5, the hand handle portion 300 includes a first connection portion 320 that is connected to the outer sheath 200 and a second connection portion 330 that is connected to at least a part of the puncture device 100 and relatively moves forward and backward with respect to the first connection portion 320 to cause the distal end of the puncture device 100 to protrude from the distal end of the outer sheath 200.

As illustrated in Figs. 3 and 4, a distal end side connection portion 310 is connected to the distal portion of the first connection portion 320.

The distal end side connection portion 310 has a connected portion 311 disposed in the vicinity of the distal portion. The connected portion 311 is configured to be connectable to the endoscope device 500. The connected portion 311 may be formed of, for example, a known luer lock type connector capable of lock-unlocking with respect to a device insertion port (not illustrated) included in the endoscope device 500.

The distal end side connection portion 310 includes a lumen 313 into which the outer sheath 200 and the puncture device 100 are inserted.

As illustrated in Figs. 3 and 4, a first stopper 360 is disposed on the distal end side of the first connection portion 320. The first stopper 360 is configured to be movable along the outer surface of the distal end side connection portion 310.

The first stopper 360 includes a fastener 361 for fixing the first stopper 360 to the distal end side connection portion 310, and a support portion 362 for supporting the fastener 361.

The fastener 361 may be formed of, for example, a screw having a male screw portion. The support portion 362 may be configured to include, for example, a female screw portion to which the fastener 361 can be screwed.

An operator who operates the medical device 10 can fix the first stopper 360 at any position in the axial direction of the distal end side connection portion 310 by screwing the fastener 361 into the support portion 362 and bringing the fastener 361 into contact with the outer surface of the distal end side connection portion 310. The operator can move the first stopper 360 along the outer surface of the distal end side connection portion 310 by operating the fastener 361 to weaken the fixing force of the first stopper 360 with respect to the distal end side connection portion 310.

The first connection portion 320 is movable forward and backward in the axial direction along the distal end side connection portion 310. The maximum forward movement position (forward movement limit position) of the first connection portion 320 in the axial direction is defined by a position where the first stopper 360 is disposed. That is, when the operator moves the first connection portion 320 in the axial direction, the operator can move the first connection portion 320 forward to a position where the distal portion of the first connection portion 320 abuts on the first stopper 360. The exposure length at which the distal end side connection portion 310 is exposed from the first connection portion 320 is adjusted by the position of the first stopper 360.

A specific configuration of the first stopper 360 is not particularly limited as long as the maximum advancing position of the first connection portion 320 can be defined as described above. The same applies to a second stopper 370 and a third stopper 380 that will be described later. In the present embodiment, the second stopper 370 and the third stopper 380 have substantially the same configuration as the first stopper 360. Therefore, in the following description, the description of the configurations of the second stopper 370 and the third stopper 380 will be omitted as appropriate.

As illustrated in Fig. 4, a large diameter portion 316 for preventing coming off is provided in the vicinity of the proximal portion of the distal end side connection portion 310. When the distal end side connection portion 310 is moved to the distal end side, the large diameter portion 316 for preventing coming off prevents the distal end side connection portion 310 from moving to the distal end side of the first stopper 360 by abutting on the support portion 362 of the first stopper 360. The medical device 10 can prevent the distal end side connection portion 310 from coming off to the distal end side with the large diameter portion 316 for preventing coming off.

As illustrated in Fig. 4, near the proximal portion of the distal end side connection portion 310, a first projection 315 that can be fitted into a first depression 327 formed on the inner wall of the first connection portion 320 is provided.

In the medical device 10, the movement of the distal end side connection portion 310 in the axial direction is restricted in a state in which the first projection 315 is fitted into the first depression 327. For example, the distal end side connection portion 310 and the first connection portion 320 can be held at a predetermined initial position by bringing the first projection 315 into a state of being fitted into the first depression 327 in a preparation stage or the like (a product shipping stage after manufacturing or a stage of storing) before the medical device 10 is used in a medical site. In the present embodiment, the first projection 315 is provided in the distal end side connection portion 310 on the axial proximal end side of the large diameter portion 316, but the large diameter portion 316 may be provided in the distal end side connection portion 310 located further toward the axial proximal end side than the first projection 315.

The first depression 327 and the first projection 315 are preferably configured to generate a fixing force that allows the first projection 315 to come out relatively easily from the first depression 327 when an operator applies a force that relatively moves one of the distal end side connection portion 310 and the first connection portion 320 in the axial direction with respect to the other. The same applies to a second depression 337 and a second projection 325 that will be described later.

As illustrated in Figs. 4 and 5, the first connection portion 320 is formed of a tubular member including a lumen 323 into which the puncture device 100 and the outer sheath 200 are inserted. A part of the proximal end side of the distal end side connection portion 310 is inserted into the lumen 323.

As illustrated in Figs. 4 and 5, the second connection portion 330 is formed of a tubular member including a lumen 333 into which the inner sheath 140 and the outer sheath 200 are inserted. A part of the proximal end side of the first connection portion 320 is inserted into the lumen 333.

As illustrated in Fig. 5, a large diameter portion 326 for preventing coming off is provided at the proximal portion of the first connection portion 320. When the first connection portion 320 is moved to the distal end side, the large diameter portion 326 for preventing coming off prevents the first connection portion 320 from moving to the distal end side of the second stopper 370 by abutting on the support portion 362 of the second stopper 370. The medical device 10 can prevent the first connection portion 320 from coming off to the distal end side with the large diameter portion 326 for preventing coming off.

As illustrated in Fig. 5, a second projection 325 that can be fitted into a second depression 337 formed on the inner wall of the second connection portion 330 is provided near the proximal portion of the first connection portion 320.

In the medical device 10, the movement of the first connection portion 320 in the axial direction is restricted in a state in which the second projection 325 is fitted into the second depression 337. The first connection portion 320 and the second connection portion 330 can be held at predetermined initial positions by bringing the second projection 325 into a state of being fitted into the second depression 337 in a preparation stage or the like before the medical device 10 is used in a medical site. In the present embodiment, the second projection 325 is provided in the first connection portion 320 located further toward the proximal end side in the axial direction than the large diameter portion 326, but the large diameter portion 326 may be provided in the first connection portion 320 located further toward the proximal end side in the axial direction than the second projection 325.

As illustrated in Fig. 5, a large diameter portion 336 for preventing coming off is provided at the proximal portion of the second connection portion 330. When the third connection portion 340 is moved toward the distal end side, the large diameter portion 336 for preventing coming off prevents the second connection portion 330 from moving toward the distal end side from the distal portion 354 of the grip portion 350 by abutting on the distal portion 354 of the grip portion 350. The medical device 10 can prevent the second connection portion 330 from coming off to the distal end side with the large diameter portion 336 for preventing coming off.

As illustrated in Figs. 4 and 5, a second stopper 370 is disposed on the distal end side of the second connection portion 330. The second stopper 370 is configured to be movable along the outer surface of the first connection portion 320.

The second connection portion 330 is movable forward and backward in the axial direction along the outer surface of the first connection portion 320. The maximum forward movement position (forward movement limit position) of the second connection portion 330 in the axial direction is defined by the position where the second stopper 370 is disposed. When the operator moves the second connection portion 330 in the axial direction, the operator can move the second connection portion 330 forward to a position where the distal portion of the second connection portion 330 abuts on the second stopper 370. In addition, the protrusion length of the inner sheath 140 connected to the first connection portion 320 protruding from the outer sheath 200 is adjusted by the position of the second stopper 370.

As illustrated in Fig. 3, at least a part of the hand handle portion 300 has a third connection portion 340. The third connection portion 340 is located further toward the proximal end side than the second connection portion 330, and is connected to the proximal portion 113 of the puncture needle 110. As illustrated in Figs. 8 and 9, the third connection portion 340 is configured to relatively move forward and backward with respect to the second connection portion 330 to cause the blade edge portion 120 to protrude from the distal end of the inner sheath 140.

The hand handle portion 300 has a grip portion 350 that can be gripped by an operator with fingers or the like. The third connection portion 340 is disposed near the proximal portion of the internal space 353 of the grip portion 350. A proximal end opening (not illustrated) located at the proximal portion 113 of the puncture needle 110 connected to the third connection portion 340 is in fluid communication with the first port 351.

The third connection portion 340 is connected to the grip portion 350 together with the first port 351. In addition, the puncture needle 110 is connected to the grip portion 350 via the third connection portion 340.

The grip portion 350 is movable forward and backward in the axial direction along the outer surface of the second connection portion 330. When the grip portion 350 moves in the axial direction along the outer surface of the second connection portion 330, the third connection portion 340 and the puncture needle 110 connected to the grip portion 350 move in the axial direction in conjunction with the movement.

Note that a protection member (protective sheath) for covering and protecting a portion of the needle tube portion 130 of the puncture needle 110 exposed from the inner sheath 140 can be appropriately disposed in an internal space 353 of the grip portion 350. In addition, a protection member (protective sheath) for covering and protecting a portion of the inner sheath 140 exposed from the outer sheath 200 can be appropriately disposed in the internal space of the lumen 333 into which the inner sheath 140 and the outer sheath 200 are inserted. A configuration of the protection member is not particularly limited as long as it is possible to prevent the puncture needle 110 from being deflected in the internal space 353 by operating forward and backward movement of the hand handle portion 300. For example, in the internal space 353 of the grip portion 350, the protection member may be a protection member that is formed of a tubular body having a small diameter and a tubular body having a large diameter that cover the outer surface 130b of the needle tube portion 130, and in which the tubular body having a small diameter of which one end is fixed to a wall on the proximal end side of the internal space 353 of the grip portion 350 can be inserted into the tubular body having a large diameter of which one end is fixed to a wall on the proximal end side of the proximal portion 146 of the inner sheath 140 connected to the second connection portion 330. Alternatively, the protection member may be formed of two tubular bodies having the same diameter that cover the outer surface 130b of the needle tube portion 130 and have engagement portions (for example, grooves or cuts) for engaging with each other, and may fix one end of each tubular body as described above and engage with each other at the engagement portions. In the internal space of the lumen 333 into which the inner sheath 140 and the outer sheath 200 are inserted, one ends of the two tubular bodies are respectively fixed to the wall on the proximal end side of the proximal portion 206 of the outer sheath 200 and the wall on the distal end side of the proximal portion 146 of the inner sheath 140 connected to the second connection portion 330. Here, the protection member may be fixed to axially penetrate the proximal portion 146 of the inner sheath 140 and the proximal portion 206 of the outer sheath 200 connected to the second connection portion 330.

As illustrated in Figs. 3 and 5, the third stopper 380 is disposed on the distal end side of the grip portion 350. The third stopper 380 is configured to be movable along the outer surface of the second connection portion 330.

The maximum forward movement position (forward movement limit position) of the grip portion 350 in the axial direction is defined by a position where the third stopper 380 is disposed. That is, when the operator moves the grip portion 350 in the axial direction, the operator can move the grip portion 350 forward to a position where the distal portion 354 of the grip portion 350 abuts on the third stopper 380. In addition, the protrusion length of the puncture needle 110 connected to the grip portion 350 via the third connection portion 340 protruding from the inner sheath 140 is adjusted by the position of the third stopper 380.

Note that the distal portion 354 of the grip portion 350 is preferably made of a resin material or a metal material having hardness enough to prevent breakage of the grip portion 350 when abutting on the third stopper 380.

As illustrated in Fig. 3, the medical device 10 has the first port 351 that is disposed to communicate with the puncture lumen 135 via the third connection portion 340 of the hand handle portion 300 and allows a fluid to be supplied into the puncture lumen 135.

The first port 351 may include, for example, a known connector to which a drug solution supply device or an intake-capable instrument can be connected.

### <Usage Example of Medical Device 10>

Next, a method of administering a drug solution using the medical device 10 will be described. The method of administering a drug solution described below is an example, and a procedure of using the medical device 10 and the like are not limited to the following content.

In the following description, a procedure of administering a drug solution to the target site E (for example, a specific region including a living tissue in which cancer cells are present) of the pancreas M using the medical device 10 will be described. Here, as illustrated in Fig. 14, a procedure of using the endoscope device 500 to deliver the medical device 10 via the stomach wall W and deliver the puncture needle 110 to the pancreas M will be described.

When starting drug solution administration using the medical device 10, an operator inserts the endoscope device 500 into a living body of a patient. After delivering the insertion portion 510 of the endoscope device 500 to a predetermined position in the stomach, the operator checks a state of the target site E by using the ultrasound imaging portion 520.

The operator connects a drug solution supply device to the first port 351 (see Fig. 3) and feeds the drug solution into the hand handle portion 300 to prime the medical device 10.

The operator operates the first stopper 360 and the distal end side connection portion 310 to adjust the effective length of the medical device 10 to correspond to the effective length of the insertion portion 510 of the endoscope device 500.

The operator inserts the medical device 10 into the channel 511 of the insertion portion 510 of the endoscope device 500 and delivers the medical device 10 to the stomach via the insertion portion 510. While the medical device 10 is being moved in the channel 511, the operator maintains a state in which the hole portion 136 is covered with the inner sheath 140 (state illustrated in Fig. 8) while causing the blade edge portion 120 of the puncture needle 110 to protrude from the inner sheath 140. In addition, while the medical device 10 is being delivered to the stomach, the operator maintains the blade edge portion 120 of the puncture needle 110 and the distal portion of the inner sheath 140 in a state of being accommodated in the first lumen 203 of the outer sheath 200 (a state in which the blade edge portion 120 of the puncture needle 110 and the distal portion of the inner sheath 140 do not protrude from the outer sheath 200).

In a state in which the puncture device 100 is covered with the outer sheath 200 as described above, the operator can deliver the medical device 10 to a desired position in the living body via the channel 511 of the endoscope device 500. In the medical device 10, an outer diameter of a portion of the medical device 10 inserted into the channel 511 is increased by an amount corresponding to the outer diameter of the outer sheath 200 compared with a case where the outer sheath 200 is not provided. Therefore, as illustrated in Fig. 6, the operator can reduce the clearance formed between the medical device 10 and the inner wall of the insertion portion 510 while moving the medical device 10 in the channel 511. Note that, in Fig. 6, the insertion portion 510 of the endoscope device 500 is illustrated by a pair of dashed lines, with the insertion portion 510 shown as the outer dashed line and the channel 511 shown as the inner dashed line.

If an excessive clearance is formed between the medical device 10 and the inner wall of the insertion portion 510 during movement of the medical device 10 in the channel 511, the medical device 10 is likely to be twisted or deflected. When the medical device 10 is twisted or deflected, the distal portion of the medical device 10 protruding from the insertion portion 510 of the endoscope device 500 protrudes from the distal end of the insertion portion 510 to be largely separated from the central axis of the channel 511. As a result, a positional deviation in the radial direction with respect to the central axis of the insertion portion 510 occurs between the proximal portion of the puncture device 100 located near the hand side of the endoscope device 500 (near the insertion port of the endoscope device 500) and the vicinity of the distal portion of the medical device 10 protruding from the insertion portion 510 of the endoscope device 500. When such a positional deviation occurs, the operability of the medical device 10 deteriorates (torque transmission is not sufficient), it is difficult for the ultrasound imaging portion 520 to ascertain the position of the blade edge portion 120 located at the distal portion of the puncture device 100, or the pushability of the puncture device 100 deteriorates, and the pushing force applied on the proximal portion side of the medical device 10 cannot be sufficiently transmitted to the blade edge portion 120. As described above, the medical device 10 according to the present embodiment can prevent generation of an excessive clearance between the medical device 10 and the inner wall of the insertion portion 510, and thus can suitably prevent the occurrence of the above-described problem.

In addition, the medical device 10 can operate protrusion of the puncture device 100 from the first lumen 203 (see Fig. 6) of the outer sheath 200 and accommodation of the puncture device 100 into the first lumen 203 of the outer sheath 200 by relatively moving the second connection portion 330 of the hand handle portion 300 forward and backward with respect to the first connection portion 320 of the hand handle portion 300 connected to the outer sheath 200. Therefore, the operator can smoothly perform a predetermined treatment (delivery of the medical device 10 to the target site E or local administration of a drug solution to the target site E) using the medical device 10.

In addition, the outer sheath 200 includes two lumens such as the first lumen 203 via which the puncture device 100 can be inserted and the second lumen 204 located radially outside the first lumen 203. The outer sheath 200 is configured to have moderate flexibility by including the two lumens 203 and 204. Thus, the operator can smoothly deliver the medical device 10 to a desired position in the living body via the channel 511 of the insertion portion 510.

As illustrated in Fig. 14, the operator can cause the puncture needle 110 and the inner sheath 140 to protrude from the insertion portion 510 via the distal end opening (not illustrated) of the insertion portion 510 by moving the medical device 10 to the distal portion of the endoscope device 500. As illustrated in Fig. 15, the operator moves the puncture device 100 toward the target site E of the pancreas M in a state in which the blade edge portion 120 of the puncture needle 110 protrudes from the inner sheath 140. In this case, the operator causes the puncture needle 110 to puncture the stomach wall W and further penetrate the stomach wall W in a state in which the hole portion 136 is covered with the inner sheath 140 (the state illustrated in Fig. 8) while causing the blade edge portion 120 of the puncture needle 110 to protrude from the inner sheath 140. By covering the hole portion 136 with the inner sheath 140 as described above, the operator can prevent the living tissue from entering the hole portion 136 and blocking (clogging) the hole portion 136 with the living tissue.

As illustrated in Fig. 16, the operator causes the blade edge portion 120 and the needle tube portion 130 to penetrate the stomach wall W and then moves the blade edge portion 120 and the needle tube portion 130 forward with respect to the inner sheath 140 to bring the portion where the blade edge portion 120 and the hole portion 136 of the needle tube portion 130 are formed into a state of protruding from the inner sheath 140 (the state illustrated in Fig. 9). The operator causes a certain range on the distal end side of the puncture needle 110 (a certain range including at least a portion located further toward the proximal end side than the position where the hole portion 136 of the needle tube portion 130 is formed) to be inserted into the target site E of the pancreas M by causing the blade edge portion 120 and the needle tube portion 130 to protrude from the inner sheath 140 as described above.

As illustrated in Fig. 17, the operator supplies the drug solution to the puncture lumen 135 via the first port 351 in a state in which the hole portion 136 of the needle tube portion 130 is disposed at the target site E. The operator can locally administer the drug solution to the target site E by releasing the drug solution supplied to the puncture lumen 135 to the outside of the needle tube portion 130 via the hole portion 136. The drug solution used in the procedure described here can be, for example, a CAR-T cell, an mRNA pharmaceutical, an anticancer agent, or an immune checkpoint inhibitor. Note that the drug solution to be administered can be selected freely depending on the content of a procedure using the medical device 10, a biological organ for which the drug solution is used, a disease to be treated, and the like.

After the administration of the drug solution to the target site E is completed, the operator removes the puncture needle 110 from the pancreas M.

The operator accommodates the puncture needle 110 removed from the pancreas M in the inner sheath 140 to obtain the state illustrated in Fig. 7. The operator accommodates the puncture needle 110 and the inner sheath 140 in the outer sheath 200 in a state in which the puncture needle 110 is accommodated in the inner sheath 140.

In a state in which the puncture needle 110 and the inner sheath 140 are accommodated in the outer sheath 200, the operator accommodates the medical device 10 in the insertion portion 510 of the endoscope device 500 and removes the medical device 10 from the living body via the channel 511 of the insertion portion 510. After the puncture needle 110 punctures the target site E, the operator performs an operation of accommodating the puncture needle 110 in the inner sheath 140 and further removing the medical device 10 to the outside of the living body in a state in which the inner sheath 140 is accommodated in the outer sheath 200, so that it is possible to prevent the living tissue (for example, the living tissue of the lesion) attached to the puncture needle 110 puncturing the target site E from being seeded in the living body.

The operator can locally administer the drug solution to the target site E using the medical device 10 according to the above procedure.

Hereinafter, functions and effects of the medical device 10 according to the present embodiment will be described.

The medical device 10 is configured as a medical device that is retractably inserted into the channel 511 of the insertion portion 510 of the endoscope device 500. The medical device 10 includes the puncture device 100, the outer sheath 200 into which the puncture device 100 is inserted, and the hand handle portion 300 disposed on the proximal end side of the puncture device 100 and the outer sheath 200. The outer sheath 200 has the first surface 201a defining the first lumen 203 into which the puncture device 100 can be inserted inside, and the second surface 201b defining at least one second lumen 204 between the first surface 201a and the second surface 201b. The hand handle portion 300 includes the first connection portion 320 connected to the outer sheath 200 and the second connection portion 330 connected to at least a part of the puncture device 100 and relatively moving forward and backward with respect to the first connection portion 320 to cause the distal end of the puncture device 100 to protrude from the distal end of the outer sheath 200.

According to the medical device 10 configured as described above, it is possible to prevent an excessive clearance from being formed with the inner wall of the endoscope device 500 (the inner wall of the insertion portion 510) when the puncture device 100 is delivered to the target site E in the living body. In addition, according to the medical device 10, the protrusion of the puncture device 100 from the first lumen 203 of the outer sheath 200 and the accommodation of the puncture device 100 into the first lumen 203 of the outer sheath 200 can be easily and smoothly performed by operating the forward and backward movement of the hand handle portion 300.

In addition, the puncture device 100 includes the puncture needle 110 including the blade edge portion 120 and the needle tube portion 130, and the inner sheath 140 including the third lumen 143 into which the puncture needle 110 is retractably inserted.

The puncture device 100 can accommodate the blade edge portion 120 of the puncture needle 110 puncturing the living tissue in the third lumen 143 of the inner sheath 140. Therefore, it is possible to prevent the living tissue (for example, the living tissue of the lesion) of the target site E attached to the puncture needle 110 from being seeded in the living body when the puncture device 100 is removed from the living body.

In addition, the inner sheath 140 is connected to the hand handle portion 300 at the second connection portion 330, and at least a part of the hand handle portion 300 is located further toward the proximal end side than the second connection portion 330, and has the third connection portion 340 to which the proximal portion 113 of the puncture needle 110 is connected, and which relatively moves forward and backward with respect to the second connection portion 330 to cause the blade edge portion 120 to protrude from the distal end of the inner sheath 140.

The puncture device 100 can easily and smoothly perform the protrusion of the inner sheath 140 from the outer sheath 200 and the accommodation of the inner sheath 140 into the outer sheath 200 by moving the second connection portion 330 to which the inner sheath 140 is connected forward and backward. In addition, the puncture device 100 can easily and smoothly perform the protrusion of the puncture needle 110 from the inner sheath 140 and the accommodation of the puncture needle 110 into the inner sheath 140 by moving the third connection portion 340 to which the puncture needle 110 is connected forward and backward.

In addition, the puncture needle 110 has the puncture lumen 135 that allows a fluid to be delivered to the distal end side.

Since the puncture lumen 135 is formed in the puncture needle 110, the medical device 10 can be suitably used for a procedure of locally administering a drug solution to the target site E.

The medical device 10 also has a first port 351 that is disposed to communicate with the puncture lumen 135 via the third connection portion 340 of the hand handle portion 300 and allows a fluid to be supplied into the puncture lumen 135.

The operator can supply various fluids (for example, a drug solution to be administered to the target site E) to the puncture lumen 135 via the first port 351. Therefore, the operator can perform a procedure of locally administering the drug solution to the target site E by using the medical device 10.

In addition, the outer sheath 200 includes the inner tube 210 having the first surface 201a and the first lumen 203, the outer tube 220 having the second surface 201b and the second lumen 204, and the fixing member 250 that is disposed between the inner tube 210 and the outer tube 220 and fixes the inner tube 210 and the outer tube 220.

In the medical device 10, since the outer sheath 200 has a multi-tube structure including a plurality of tubular members including the inner tube 210 and the outer tube 220, kink resistance and breakage resistance of the outer sheath 200 can be enhanced. In addition, since the space-shaped second lumen 204 partitioned by the fixing member 250 is formed between the inner tube 210 and the outer tube 220, it is possible to prevent the rigidity of the outer sheath 200 from being excessively increased, and to impart appropriate flexibility to the outer sheath 200.

The inner tube 210 and the outer tube 220 are concentrically disposed by the fixing member 250.

In the outer sheath 200, since the inner tube 210 and the outer tube 220 are concentrically disposed by the fixing member 250, kink resistance and breakage resistance in the outer circumferential direction of the outer sheath 200 can be uniformly enhanced. Therefore, it is possible to more reliably prevent the outer sheath 200 from being damaged or the like while the medical device 10 is moved in the channel 511 of the insertion portion 510 of the endoscope device 500. In addition, since the second lumen 204 is partitioned in a uniform size in the outer circumferential direction of the outer sheath 200, the flexibility of the second lumen 204 can be made uniform in each portion in the outer circumferential direction. Therefore, since the outer sheath 200 can be deformed to follow a bent portion in the living body, the operability of the medical device 10 is improved.

Next, modifications of the above-described embodiment will be described. In the description of the modifications, the description of the procedure of using the member or the medical device, which has already been described, will be omitted as appropriate. Furthermore, contents that are not particularly described in the modifications may be the same as those in the above-described embodiment.

### <Modification 1>

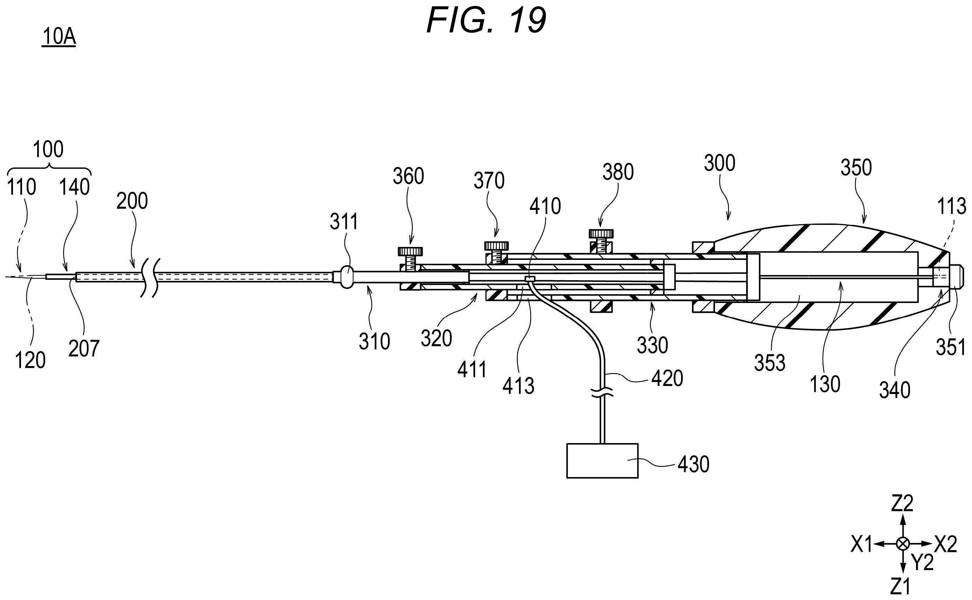

Fig. 19 illustrates a medical device 10A according to Modification 1. Fig. 19 is a partial cross-sectional view in an axial direction of the medical device 10A corresponding to Fig. 3 illustrated in the above-described embodiment.

As illustrated in Fig. 19, in the medical device 10A according to Modification 1, the first connection portion 320 includes a second port 410 that is disposed to communicate with the inside of the first lumen 203 (see Fig. 6) of the outer sheath 200 and enables fluid communication inside and outside the first lumen 203.

The second port 410 may include, for example, a connector portion connected to a predetermined tube 420 in a liquid-tight/airtight manner.

Each of the first connection portion 320 and the second connection portion 330 may be provided with hole portions 411 and 413 for preventing interference of the tube 420 when the first connection portion 320 and the second connection portion 330 move in the axial direction.

The operator can connect the second port 410 and a predetermined fluid device 430 (for example, a suction pump capable of generating a negative pressure) via the tube 420 in a procedure using the medical device 10. When the puncture needle 110 punctures a living tissue (see Figs. 15 and 16), the operator can fix the distal portion of the outer sheath 200 to biological organ to be punctured by operating the fluid device 430 to generate negative pressure in the first lumen 203. The operator can more reliably puncture the biological organ with the blade edge portion 120 of the puncture needle 110 with respect to a desired puncture position by causing the puncture needle 110 to protrude from the outer sheath 200 in a state in which the distal portion of the outer sheath 200 is fixed to the biological organ to be punctured.

The second port 410 may be disposed to enable fluid communication inside and outside the first lumen 203 and inside and outside the second lumen 204. By generating a negative pressure in the second lumen 204, the operator can fix the distal portion 354 of the outer sheath 200 to a biological organ or the like to be punctured, similarly to when the negative pressure is generated in the first lumen 203.

### <Modification 2>

Fig. 20 is a perspective view of an outer sheath 200A according to Modification 2. Fig. 21 is an axis-orthogonal cross-sectional view of the outer sheath 200A along the arrows 21A-21A illustrated in Fig. 20.

As illustrated in Figs. 20 and 21, the fixing member 450 may be configured in a shape extending spirally around the central axes C1 and C2 of the inner tube 210 and the outer tube 220 of the outer sheath 200A.

As illustrated in Fig. 21, the cross-sectional shape of the fixing member 450 can be, for example, a circular shape. However, the cross-sectional shape of the fixing member 450 is not particularly limited, and may be, for example, a rectangular shape or an elliptical shape.

Similarly to the above-described embodiment, the fixing member 450 is preferably made of a relatively flexible resin material from the viewpoint of suppressing an excessive increase in rigidity of the outer sheath 200.

The fixing member 450 may be formed of, for example, one member or two or more members. Further, the spiral shape (the width of the gap in the axial direction, and the like) of the fixing member 450 is also not particularly limited.

In the outer sheath 200A according to Modification 2, since the fixing member 450 extends spirally around the central axes C1 and C2 of the inner tube 210 and the outer tube 220, rigidity in each portion in the axial direction of the outer sheath 200A is uniform. In addition, as illustrated in Fig. 21, in each portion in the axial direction of the outer sheath 200A, since a range (occupied area on the axis-orthogonal cross-sectional view) in which the fixing member 450 exists between the inner tube 210 and the outer tube 220 is small, the flexibility of the outer sheath 200A is improved. Therefore, the outer sheath 200A is further improved in followability to a bent portion and the like in the living body.

### <Modification 3>

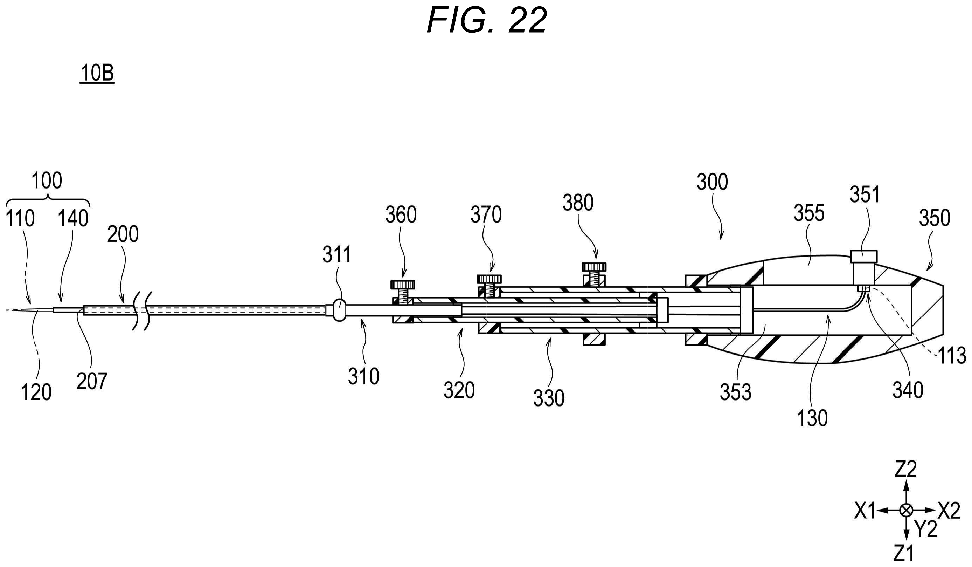

Fig. 22 illustrates a medical device 10B according to Modification 3. Fig. 22 is a partial cross-sectional view in an axial direction of the medical device 10B corresponding to Fig. 3 illustrated in the above-described embodiment.

As illustrated in Fig. 22, the third connection portion 340 may be configured to be movable forward and backward along a slide hole 355 formed in the grip portion 350, for example. The third connection portion 340 is movable forward and backward in the extending direction of the grip portion 350 which is the same direction as the axial direction of the medical device 10B by being forward and backward along the slide hole 355. The puncture needle 110 connected to the third connection portion 340 moves in the axial direction of the medical device 10B in conjunction with the movement of the grip portion 350.

Note that the first port 351 disposed to partially protrude can be connected to the third connection portion 340 via the slide hole 355. The operator can move the third connection portion 340 and the puncture needle 110 forward and backward by gripping and operating the first port 351 with fingers or the like.

Although the medical device according to the present invention has been described above through the embodiments and the modifications, the present invention is not limited to only the configurations described in the embodiments and the modifications, and can be changed as appropriate on the basis of the description of the claims.

The medical device may be configured as a device intended only for puncturing a living tissue. When the medical device is configured in such a manner, the puncture needle need not be provided with the puncture lumen. The hand handle portion need not include the first port.

The outer sheath need not be formed of two tubes (inner tube and outer tube). That is, the outer sheath may be formed of one hollow member in which the first lumen and the second lumen are formed. When the outer sheath is configured in this manner, the fixing member may be omitted.

The delivery device is not particularly limited to a product specification or a specific structure as long as the delivery device includes a passage (lumen) into which the medical device is inserted to be movable forward and backward. For example, the delivery device may be a catheter or the like including a lumen into which the medical device is inserted to be movable forward and backward.

The content of the procedure to which the medical device is applied, a biological organ to be applied, a disease, and the like are not particularly limited, and are not limited to the content described in the specification.

The present application is based on Japanese Patent Application No. 2023-169994 filed on September 29, 2023, the disclosure content of which is incorporated herein by reference in its entirety.

### Reference Signs List

- 10: Medical device
- 10A: Medical device
- 10B: Medical device
- 100: Puncture device
- 110: Puncture needle
- 120: Blade edge portion
- 121: Blade surface
- 123: Ridge line
- 130: Needle tube portion
- 135: Puncture lumen
- 136: Hole portion
- 140: Inner sheath
- 143: Third lumen
- 200: Outer sheath
- 200A: Outer sheath
- 201a: First surface
- 201b: Second surface
- 203: First lumen
- 204: Second lumen
- 210: Inner tube
- 220: Outer tube
- 250: Fixing member
- 300: Hand handle portion
- 310: Distal end side connection portion
- 320: First connection portion
- 330: Second connection portion
- 350: Grip portion
- 351: First port
- 360: First stopper
- 370: Second stopper
- 380: Third stopper
- 410: Second port
- 450: Fixing member
- 460: Lock mechanism
- 470: Protection sheath
- 500: Endoscope device (delivery device)
- 510: Insertion portion
- 511: Channel
- 520: Ultrasound imaging portion
- C1: Central axis of inner sheath
- C2: Central axis of outer sheath
- C3: Central axis of puncture needle
- g: Clearance
- E: Target site
- M: Pancreas
- W: Stomach wall

## Claims

1. A medical device that is retractably inserted into a delivery device, the medical device comprising:
a puncture device;
an outer sheath into which the puncture device is inserted; and
a hand handle portion disposed on a proximal end side of the puncture device and the outer sheath, wherein
the outer sheath includes a first surface defining a first lumen into which the puncture device is insertable inside, and a second surface defining at least one second lumen between the first surface and the second surface, and
the hand handle portion includes a first connection portion connected to the outer sheath, and a second connection portion connected to at least a part of the puncture device, and relatively moving forward and backward with respect to the first connection portion to cause a distal end of the puncture device to protrude from a distal end of the outer sheath.

2. The medical device according to claim 1, wherein the puncture device includes a puncture needle including a blade edge portion and a needle tube portion, and an inner sheath including a third lumen into which the puncture needle is retractably inserted.

3. The medical device according to claim 2, wherein the inner sheath is connected to the hand handle portion at the second connection portion, and
at least a part of the hand handle portion further includes a third connection portion that is located further toward the proximal end side than the second connection portion, to which a proximal portion of the puncture needle is connected, and that relatively moves forward and backward with respect to the second connection portion to cause the blade edge portion to protrude from a distal end of the inner sheath.

4. The medical device according to claim 3, wherein the puncture needle has a puncture lumen configured to allow a fluid to be delivered to a distal end side of the puncture needle.

5. The medical device according to claim 4, further comprising a first port that is disposed to communicate with the puncture lumen via the third connection portion of the hand handle portion and allows a fluid to be supplied into the puncture lumen.

6. The medical device according to claim 1, wherein the first connection portion includes a second port in communication with an inside of the first lumen of the outer sheath and configured to enable fluid communication inside and outside the first lumen.

7. The medical device according to claim 1, wherein the outer sheath includes
an inner tube having the first surface and the first lumen,
an outer tube having the second surface and the second lumen, and
a fixing member disposed between the inner tube and the outer tube and fixing the inner tube and the outer tube.

8. The medical device according to claim 7, wherein the inner tube and the outer tube are concentrically disposed by the fixing member.

9. The medical device according to claim 8, wherein the fixing member extends spirally around central axes of the inner tube and the outer tube.
